# EUROPEAN PATENT APPLICATION

(11) **EP 4 606 786 A1**
(43) Date of publication of application: **27.08.2025**
(21) Application number: 24158804.5
(22) Date of filing: 21.02.2024
(51) Int. Cl.: C07C 209/62, C07C 209/84

(54) **EXTRACTION OF 1,6-HEXAMETHYLENEDIAMINE USING COMBINATIONS OF SOLVENTS**

(71) Applicant: Covestro Deutschland AG, 51373 Leverkusen (DE)
(72) Inventor: Krause, Jonas, 51373 Leverkusen (DE); Altuntepe, Emrah, 51373 Leverkusen (DE); Faecke, Thomas, 51373 Leverkusen (DE); Gamez, Jose, 51373 Leverkusen (DE); Waltermann, Thomas, 51373 Leverkusen (DE)
(74) Representative: Levpat

(57) **Abstract**

The present invention relates to the extraction of 1,6-hexamethylenediamine from aqueous mixtures using combinations of solvents.

## Description

The present invention relates to the extraction of 1,6-hexamethylenediamine from aqueous mixtures using combinations of solvents.

### Technical Background

Depolymerization of polyamides and certain polyurethanes results aqueous solutions comprising diamines. Different methods for the removal of diamine from water are described in the technical field. An efficient way to remove the diamine from the water and water soluble impurities is the extraction of the diamine using monovalent alcohols followed by distillation. Since diamines, such as 1,6-hexamethylenediamine, are highly polar, their separation with a water-non-miscible organic solvent is not easy and thus the selection of a proper organic solvent is difficult.

It is known that monovalent solvents are selective and sufficient solvents, but due to the alcohols physicochemical properties the removal of water from the diamine is limited and a significant amount of water is transferred to the organic extract phase and needs to be distilled off. This increases energy demand of the process and reduces advantages of the extraction process.

In order to minimize the energy consumption during distillation it is desirable to use a solvent with low polarity. However, diamines, such as 1,6-hexamethylenediamine, themselves are regularly very polar and the partition coefficient decreases with decreasing polarity of the solvent.

EP 2 806 026 A1 relates to the production of 1,5-pentamethylenediamine. Within this process, a separation step from an aqueous solution is described. This separation can be conducted as an extraction process. The extractant can be an organic solvent, preferably an aliphatic alcohol.

US 2017/0369913 A1 describes the extraction of 1,6-hexamethylenediamine with organic solvents from aqueous solutions. Preferably, alcohols are used. However, also alkanes are tested and found to extract only little amounts of 1,6-hexamethylenediamine but also only little amounts of water. It is not disclosed that a combination of alkanes and alcohols is more advantageous than the use of alcohols or alkanes as single compounds.

### Technical objective

The problem of the present invention was to provide an improved method for extracting 1,6-hexamethylenediamine from aqueous mixtures that offers economic and as far as possible also ecological benefits compared to the state of the art.

### Technical Solution

The technical objective can be solved by a method for separating 1,6-hexamethylenediamine (HMDA)from an aqueous solution, comprising the steps:
a) providing an HMDA-containing aqueous solution by depolymerizing an HMDA-based polymer,
b) extracting the HMDA-containing aqueous solution with a solvent mixture comprising
   (i) at least one monovalent alcohol having 4 to 7 carbon atoms and
   (ii) at least one alkane having 5 to 7 carbon atoms, wherein the alkane content of the solvent mixture is in the range from 2 wt.-% to 50 wt.-% based on the total weight of all alcohols and alkanes present in the solvent mixture;
c) obtaining an HMDA-containing solvent mixture as separate phase, and
d) distilling the HMDA-containing solvent mixture and separating HMDA from the solvent mixture.

It was found that using a specific mixture of solvents is advantageous for the extraction of HMDA. The first solvent is a monovalent alcohol having 4 to 7 carbon atoms, preferably 1-butanol, 1-pentanol, 1-hexanol, 1-heptanol, cyclopentanol or cyclohexanol. As a second solvent an alkane having 5 to 7 carbon atoms is added, preferably a n-alkane.

Generally, to reduce the energy demand for distillation the amount of water in the organic extract phase can be reduced by modifying the organic solvent properties. To modify the organic solvent properties additives can be introduced, as for example non-polar solvent like alkanes. However, diamines, such as 1,6-Hexamethylenediamine, are highly polar.

Therefore, it was expected that the partitioning into the modified organic solvent phase would be worse. Surprisingly it was found that the modification of the organic extract phase by adding additives leads not only to a reduction of water and energy demand for distillation but also leads to an improved partitioning, if selected carefully. Even in cases, where the partition coefficient does not increase, the water content of the mixture of organic solvents and HMDA is decreased leading to a decreased energy consumption during subsequent distillation. This effect has to be balanced with a decreasing HMDA-concentration in the organic phase which increases energy consumption.

### Advantageous technical effect

Firstly, a lower water-content in the extract can be achieved. Secondly, a surprisingly increased partition coefficient can be observed. As set forth above, this technical effect of a unipolar solvent on the extraction of a polar compound was unexpected.

### Detailed description of the invention

In accordance with the invention, the expressions "comprising", "containing" or "including" preferably mean "consisting substantially of" and particularly preferably "consisting of".

Statements concerning the content of organic compounds in the context of the present invention refer, unless stated otherwise, to values determined by gas chromatography. The skilled person is familiar with the quantitative evaluation of gas chromatograms, optionally using an internal standard. The skilled person is likewise familiar with any methods that might perhaps be needed to determine the water content. The methods known in the art can also be used in the context of the present invention. In case of doubt, the water content determined by Karl Fischer titration is decisive. The presence of amines can lead to trailing end points. In such cases, the figure determined by Karl Fischer titration after buffering with anhydrous benzoic acid is decisive. The skilled person is likewise familiar with the method using buffering with benzoic acid. For Karl Fischer titration in general, see Jander, Jahr, Massanalyse, 17th ed., de Gruyter, Berlin (2009), p. 279 to p. 282).

"Distillation" in the present case is understood to mean a thermal separation process used to recover evaporable substances, preferably liquids, from a composition. The separated vapors are subsequently condensated. The term encompasses in particular repeated evaporation and condensation using a column (distillation column) with a plurality of separating stages. Processes of this kind in which several distillation steps are arranged in series in a column should strictly speaking be called rectification, but will nevertheless likewise be referred to herein as distillation for the purpose of simplification. The advantage of these processes is the powerful separating effect and the possibility of operating the plant continuously. A "distillation apparatus" is accordingly an apparatus which is suitable for carrying out such a thermal separation process, i.e. for example sieve plate columns, packed columns, packed-bed columns, bubble cap tray columns or even single-stage evaporators such as falling film evaporators, thin-film evaporators, flash evaporators, multi-phase helical-coil evaporators, natural or forced circulation evaporators.

The relevant pressure of a distillation apparatus in the present case is the respective head pressure prevailing in the device, i.e. the pressure at which the vapors are present before they exit the distillation apparatus. This pressure will therefore also be referred to below as head pressure.

The individual process steps will be explained in more detail below.

### Step a)

In the first step a) of the method of the invention, an HMDA-containing aqueous solution is provided by depolymerizing an HMDA-based polymer or a mixture of polymers containing an HDMA-based polymer. The polymer may be coated or may comprise an organic or inorganic filler. The content of HMDA can be freely chosen to allow efficient extraction of the HMDA. The aqueous composition comprises at least HMDA and water. Typically, it will comprise further depolymerization products of the polymer. In case of depolymerization of polyamides obtained from HMDA and a dicarboxylic acid, in particular polyamide 6.4, 6.6, 6.9, 6.10 and 6.12, such an additional depolymerization product may be the respective dicarboxylic acid. Further, the aqueous composition may comprise residues of the of catalysts and/or additives used for the depolymerization reaction, particularly salts. It may also comprise residues of any material which was used as a coating or filler of the original polymer.

In one preferred embodiment, the additional depolymerization product is separated from the aqueous composition in method step a) so that the composition present at the beginning of method step b) comprises HMDA, residues of the of catalysts and/or additives used for the depolymerization reaction but no further depolymerization products.

In a further preferred embodiment of the present invention, method step a) comprises a further sub-step of decreasing the water content of the composition after depolymerization. This sub-step may be performed in addition to the separation of additional depolymerization products or as an alternative thereto. A preferred method is the partial removal of water using a distillation as described in PCT/EP2022/075933.

Independently of the additional constituents of the aqueous composition comprising HMDA at the end of method step a) the composition comprises 0.1 to 50 wt.-% HMDA, preferably 5 to 20 wt.-% HMDA and most preferably 7 to 22 wt.-% HMDA. The water content of the composition is preferably at least 50 wt.-%, more preferably at least 55 wt.-%. All percentages are based on the total weight of the composition as 100 wt.-%. In addition, the HMDA-containing aqueous solution usually also contains other substances apart from water which have a lower boiling point than HMDA. The content of these substances which have a lower boiling point than the amine is preferably ≤ 10 wt.-%, particularly preferably ≤ 5 wt.-% and most particularly preferably ≤ 3 wt.-%. At the same time, the content in technical processes is usually ≥ 0.01 wt.-%, preferably ≥ 0.3 wt.-%, unless low boilers have already been separated beforehand. Additionally, the HMDA-containing aqueous solution usually also contains other substances which have a higher boiling point than the amine. The proportion of these substances which have a higher boiling point than the amine in the first composition preferably totals ≥0.01 wt.-% and ≤10 wt.-%, particularly preferably ≥0.1 wt.-% and ≤5 wt.-%, and most particularly preferably ≥0.3 wt.-% and ≤3 wt.-%.

The term "HMDA-based polymer" refers to all polymers that can release HMDA as single molecule using the appropriate chemical reactions. Depending on the type of polymer, additional compounds may be released as additional depolymerization products. For polyamides obtained by reacting HMDA and a dicarboxylic acid the additional depolymerization product will be the respective dicarboxylic acid, its salt or a derivative of this acid. Preferred "HMDA-based polymers" are polyamides and polyurethanes. Preferred polyamides are polyamide 6.4, polyamide 6.6, polyamide 6.9 and polyamide 6.10. A particularly preferred "HMDA-based polymer" is polyamide 6.6 which is the reaction product of adipic acid and HMDA.

The appropriate chemical reaction which releases HMDA from a polymer obviously depends on the type of bonds present in the polymer. In case of polyamides such chemical reaction needs to cleave the amide bond so that HMDA is released. In case of polyamides, it is preferred to cleave the amide bond by hydrolysis
Preferred methods for the hydrolysis of polyamides are ammonolysis, acidic hydrolysis, alkaline hydrolysis, glycolysis, hydrolysis by microwaves and enzymatic hydrolysis. Amongst these acidic and alkaline hydrolysis are preferred with particular preference for alkaline hydrolysis.

Preferred agents for acidic hydrolysis are aqueous solutions of HCl, formic acid, sulphuric acid and nitric acid. Suitable reaction temperatures for acidic hydrolysis are in the range between 80 and 120 °C. Acidic hydrolysis is preferably performed at a pressure between 1 and 6 bar. Suitable methods for acidic hydrolysis are described in US 3,069,465 and EP 0 833 809.

Preferred agents for alkaline hydrolysis are alkali hydroxide solutions in water or mixtures of water and alcohols. Preferred alkali hydroxides are NaOH and KOH. Particularly preferred are aqueous solutions of NaOH and KOH. Suitable reaction temperatures for alkaline hydrolysis are in the range between 140 and 220 °C. Alkaline hydrolysis is preferably performed at a pressure between 0.8 and 150 bar. Suitable methods for alkaline hydrolysis are described in EP 0 646 106 or US 2,840,606.

Ammonolysis is preferably performed at temperatures between 300 and 350 °C and pressures between 34 and 172 bar. A suitable method is described in US 5,302,756.

It is to be understood that method steps a) and b) may be performed at different locations and/or with a time lag. In one preferred embodiment of the present invention, the distance between the location, where method step b) is performed and the location, where method step b) is performed, is at least 1 km, preferably at least 5 km, more preferably at least 10 km and most preferably at least 20 km. In another preferred embodiment of the present invention, the time lag between the end of method step a) and the commencement of method step b) is at least 1 hour, preferably at least 12 hours, more preferably at least 24 hours and most preferably at least 72 hours.

In certain embodiments of the present invention, method step a) may be preceded by a method step by breaking a polymer article into smaller particles so that the surface to volume ratio of the polymer increases, thus facilitating depolymerization. This can be achieved by all suitable methods known to person skilled in the art.

Substances with lower boiling points compared to HMDA that can occur in the HMDA-containing aqueous solution are, apart from water, for example ammonia, alkyl or alkenyl amines, alcohols, ethylene glycol, cyclic amines such as pyrrolidine, piperidine, azepane, imines, cylic imines such as tetrahydropyridine or tetrahydroazepine, or residues of solvents such as hydrocarbons or halogenated hydrocarbons for example. In addition, gases such as nitrogen or carbon dioxide may be present in the solution in dissolved or bound form.

Substances with higher boiling points compared to HMDA that can occur are for example dimers or oligomers of HMDA, which can form while cleaving off ammonia from HMDA.

An HMDA-containing aqueous solution originating from the chemical recycling of polyamides, such as PA66, may also contain adipic acid, salts thereof or fragments of PA66 as substances with higher boiling points. Statements concerning relative contents refer, unless stated otherwise, to the totality of the substances with higher boiling points compared to HMDA.

The HMDA present in the solution provided in method step a) is preferably present in uncharged form. This is typically the case at a pH of at least 11.5. Therefore, the HMDA-containing aqueous solution in method step a) preferably has a pH of at least 11.5, more preferably 12.0.

### Step b)

In the second step b) of the method of the invention, the HMDA-containing aqueous solution is extracted with a solvent mixture comprising (i) at least one monovalent alcohol having 4 to 7 carbon atoms; and (ii) at least one alkane having 5 to 7 carbon atoms, wherein the alkane content of the solvent mixture is in the range from 2 wt.-% to 50 wt.-% based on the total weight of all alcohols and alkanes present in the solvent mixture.

Preferably, the at least one monovalent alcohol having 4 to 7 carbon atoms is selected from the group consisting of 1-butanol, 1-pentanol, 1-hexanol, 1-heptanol, cyclopentanol, cyclohexanol and mixtures thereof. More preferably, the at least one monovalent alcohol having 4 to 7 carbon atoms is selected from the group consisting of 1-butanol, 1-pentanol and 1-hexanol and mixtures thereof. Most preferably, the at least one monovalent alcohol having 4 to 7 carbon atoms is 1-hexanol.

The at least one alkane having 5 to 7 carbon atoms may be linear, branched or cyclic. More preferably, the at least one alkane having 5 to 7 carbon atoms may be linear or cyclic. Most preferably, the at least one alkane having 5 to 7 carbon atoms may be linear.

Preferably, the at least one alkane having 5 to 7 carbon atoms is selected from n-pentane, n-hexane, n-heptane and mixtures thereof.

Preferably, the alkane content of the solvent mixture is in the range from 2 wt.-% to 40 wt.-%, more preferably 2 wt.-% to 30 wt.-%, most preferably 2 wt.-% to 20 wt.-% based on the total weight of all alcohols and alkanes present in the solvent mixture.

### Step c)

In the third step c) of the method of the invention, an HMDA-containing solvent mixture is obtained as separate phase. The measures in order to obtain the HMDA-containing solvent mixture as separate phase are not particularly limited. In general, the HMDA-containing solvent mixture can be separated from the aqueous phase after step b) as soon as two distinct phases are formed. The process can be performed in a mixer-settler, extraction column or centrifugal separator with 1 to 20 stages, preferably 3 to 15 stages, most preferably 5 to 10 stages.

### Step d)

In the fourth step d) of the method of the invention, the HMDA-containing solvent mixture is distilled and HMDA is separated from the solvent mixture.

A distillation device used in step d) is not limited, but any distillation device known in the art for such separation steps may be used. The distillation device may be a single distillation column. The single distillation column may have 5 to 100, 10 to 60 or 20 to 50 theoretical stages. In case, 1-hexanol is the monovalent alcohol, 20 or more theoretical stages, e.g., 20 to 50 theoretical stages, may be used. The distillation pressure may be from 100-1,000 mbar, 150-800 mbar, 150-700 mbar, 200-600 mbar, 200-500 mbar or 200-400 mbar. It is, thus, lower than the atmospheric pressure of 1,013 mbar.

Any combinations of embodiments, especially preferred, more preferred, most preferred ranges and/or embodiments are suitable and selectable and are particularly preferred.

### Brief description of the drawings

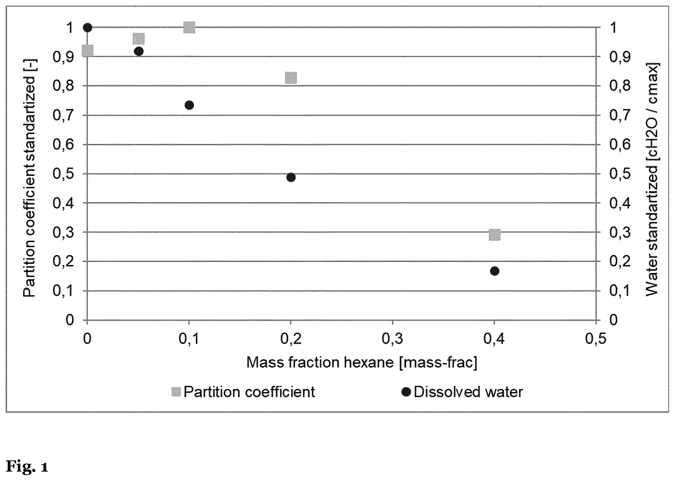

Fig. 1 is a graph illustrating the dependency of the partition coefficient and the water content of the solvent mixture on the proportion of butanol and hexane as analyzed in example 1;

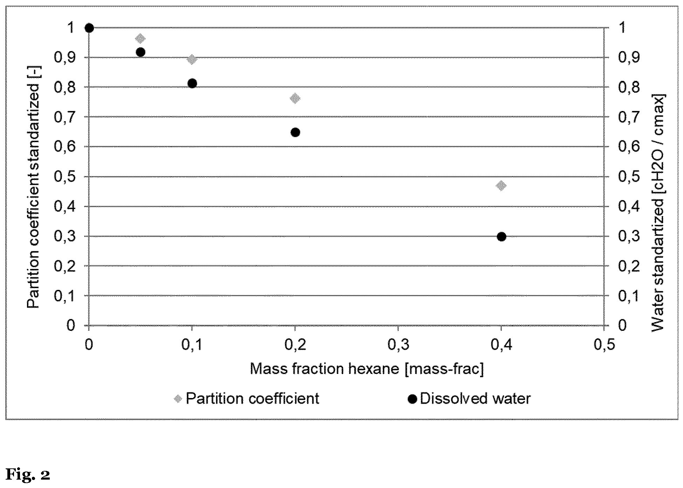

Fig. 2 is a graph illustrating the dependency of the partition coefficient and the water content of the solvent mixture on the proportion of hexanol and hexane as analyzed in example 2.

The present invention will be described in more detail in the following examples. However, these examples are for illustrative purposes only.

### Examples

### Example 1: Extraction of HMDA using 1-Butanol

The aqueous phase was synthetically created by dissolving 10 wt.-% of HMDA in 1 molar aqueous NaOH. The organic phase was created by mixing various concentrations of n-hexane and 1-butanol. The organic phase was saturated with water before used for extraction as the organic phase. 100 g of the aqueous solution was mixed with 35 g of the water saturated organic phase and the temperature was adjusted to 50 °C. Mixing was then continued for at least 10 minutes to reach equilibrium. Afterwards the phases were settled by gravity until both phases were completely separated. The concentration of HMDA was measured via HPLC-MS in both phases. For evaluation the partition coefficient was calculated via the concentration in the organic phase over the concentration of HMDA in the aqueous phase and standardized to the highest value measured. In addition, the dissolved water was measured via Karl-Fischer titration in the organic alcohol/alkane mixture and standardized to the highest value measured.

A mass fraction of hexane in the organic phase in the range of 5 to 10 wt.-% increases the partition coefficient. Moreover, the water content of the organic phase decreased with increasing concentrations of hexane.

The aqueous phase was synthetically created by dissolving 10 wt.-% of HMDA in 1 molar aqueous NaOH. The organic phase was created by mixing various concentrations of n-hexane and 1-hexanol. The organic phase was saturated with water before used for extraction as the organic phase. 100 g of the aqueous solution was mixed with 35 g of the water saturated organic phase and the temperature was adjusted to 50 °C. Mixing was then continued for at least 10 minutes to reach equilibrium. Afterwards the phases were settled by gravity until both phases were completely separated. The concentration of HMDA was measured via HPLC-MS in both phases. For evaluation the partition coefficient was calculated via the concentration in the organic phase over the concentration of HMDA in the aqueous phase and standardized to the highest value measured. In addition, the dissolved water was measured via Karl-Fischer titration in the organic alcohol/alkane mixture and standardized to the highest value measured.

With 1-hexanol the addition of hexane did not increase the partition coefficient. Nevertheless, the decreased water content of the organic phase concomitantly decreases the energy requirements during separation of the HMDA from the solvents during distillation as long as it is not too high. Generally, a mass fraction of hexane of 40 wt.-% is the limit, where the decreased energy consumption due to decreased water content of the organic phase is offset by the increased energy consumption due to the need to remove too much organic solvent.

## Claims

1. Method for separating 1,6-hexamethylenediamine (HMDA) from an aqueous solution, comprising the steps:
a) providing a diamine-containing aqueous solution by depolymerizing an HMDA-based polymer,
b) extracting the HMDA-containing aqueous solution with a solvent mixture comprising
(i) at least one monovalent alcohol having 4 to 7 carbon atoms and
(ii) at least one alkane having 5 to 7 carbon atoms,
wherein the alkane content of the solvent mixture is in the range from 2 wt.-% to 50 wt.-% based on the total weight of all alcohols and alkanes present in the solvent mixture;
c) obtaining an HMDA-containing solvent mixture as separate phase, and
d) distilling the HMDA-containing solvent mixture and separating the HMDA from the solvent mixture.

2. The method according to claim 1, wherein the at least one monovalent alcohol having 4 to 7 carbon atoms is selected from the group consisting of 1-butanol, 1-pentanol, 1-hexanol, 1-heptanol, cyclopentanol, cyclohexanol and mixtures thereof.

3. The method according to claim 1 or 2, wherein the at least one alkane having 5 to 7 carbon atoms is a linear alkane.

4. The method according to any of the preceding claims, wherein the alkane content of the solvent mixture is in the range from 2 wt.-% to 40 wt.-% based on the total weight of all alcohols and alkanes present in the solvent mixture.

5. The method according to any of the preceding claims, wherein the alkane content of the solvent mixture is in the range from 2 wt.-% to 20 wt.-% based on the total weight of all alcohols and alkanes present in the solvent mixture.

6. The method according to any of the preceding claims, wherein the aqueous composition comprises 0.1 to 50 wt.-% HMDA based on the total weight of the aqueous composition.

7. The method according to any of the preceding claims, wherein the HMDA-based polymer is a polyamide obtained by (i) condensation of HMDA and a dicarboxylic acid or (ii) a polyurethane.

8. The method according to claim 7, wherein the polyamide is selected from the group consisting of polyamide 6.4, polyamide 6.6, polyamide 6.9 polyamide 6.10 and polyamide 6.12.

9. The method according to claim 7 or 8, wherein depolymerization of the polyamide is achieved by a method selected from the group consisting of acidic hydrolysis, alkaline hydrolysis, ammonolysis, glycolysis, hydrolysis by microwaves and enzymatic hydrolysis.

10. The method of any one of the preceding claims, wherein method step a) and method step b) are performed at different locations with a minimum distance of 1 km.
